# EUROPEAN PATENT APPLICATION

(11) **EP 0 947 821 A1**
(43) Date of publication of application: **06.10.1999**
(21) Application number: 99106001.3
(22) Date of filing: 25.03.1999
(51) Int. Cl.: G01N 5/00, G01N 9/26, G01N 33/14, C12G 1/02, C12M 1/36

(54) **Device for continuously detecting the development of reaction processes in a liquid**

(30) Priority: 30.03.1998 IT MI980663
(71) Applicant: Plasmati Eustachio, 20090 Segrate (MI) (IT)
(72) Inventor: Plasmati Eustachio, 20090 Segrate (MI) (IT)
(74) Representative: Forattini, Amelia

(57) **Abstract**

A device for continuously detecting the development of reaction processes, in a liquid, includes a tank (5) containing the liquid and a weight sensor (9) for detecting the weight of the liquid. The weight sensor is associated with a unit for processing the measurement of the weight and which is suitable to determine the weight variation over time in order to control the development of the reaction process which causes a change in the weight of the liquid (7).

## Description

The present invention relates to a device for continuously detecting the development of reaction processes in a liquid.

More particularly, the present invention is described in relation to the process for converting sugars into alcohol by yeasts in a sugary solution known as must. The invention, however, can be used advantageously in any process for converting a liquid contained in a tank when the process causes a decrease in the weight of the liquid.

The conversion of sugar into alcohol is an exothermic reaction which releases carbon dioxide and entails a consequent weight loss. In order to achieve the best quality level of the final product, it is necessary to control both the fermentation rate and the temperature of the liquid, bearing in mind that the optimum value of these two factors varies in the various steps of the fermentation process.

The current method detects the temperature of the liquid in one point of the tank and action is taken, on the basis of the measured value, by using heat exchangers, to contain the temperature within the preset limits.

The fermentation rate is detected by taking samples which are subjected to chemical analysis or physical measurement (for example by means of refractometers) of the residual sugar content.

Accordingly, continuous monitoring of the tank and considerable analysis work are necessary in order to constantly control the development of fermentation and to be able to act promptly.

The aim of the present invention is to provide a device for detecting the development of reaction processes in a liquid which overcomes the drawbacks of the known art.

An object of the invention is to provide a device for detecting the development of reaction processes in a liquid which allows continuous and real-time detection.

Another object is to provide a device which is constructively simple and reliable.

This aim, these objects and others which will become apparent hereinafter are achieved by a device for continuously detecting the development of reaction processes in a liquid, which includes a tank which contains a liquid and is characterized in that it includes a means for detecting the weight of the liquid which is associated with a unit for processing the measurement of the weight which is suitable to determine the weight variation over time in order to control the development of a reaction process which causes a change in the weight of the liquid.

Further characteristics and advantages will become apparent from the description of a preferred but not exclusive embodiment of the invention, illustrated only by way of non-limitative example in the accompanying drawings, wherein:
Figure 1 is a schematic lateral elevation view of the device for continuously detecting the development of reaction processes in a liquid, according to the present invention;
Figure 2 is a partially sectional lateral elevation view of the device according to the invention, applied to a tank;
Figure 3 is a schematic lateral elevation view of a tank provided with the device according to the invention.

With reference to the above figures, the device for continuously detecting the development of fermentation processes, according to the invention, generally designated by the reference numeral 1, includes a tubular body 3 associated with a tank 5 containing a liquid 7.

A pressure detection means, for example a pressure transducer 9, is applied to the tubular body 3 so as to form an air pocket 15 having a height h between the sensor 9 and the surface of the liquid 7.

Advantageously, the tubular body 3 is provided with a coupling 11 for a valve 13 which allows to introduce a jet of washing fluid, for example water, adapted to wash the portion of tubular body affected by the liquid or must to be measured.

The position of the valve 13 and of the coupling 11 are such that the jet of washing fluid does not strike the pressure sensor 9 and damage it.

The pressure sensor is preferably arranged at the base of the tank and is connected to a processing unit (not shown), which is typically constituted by a programmable controller or by a PC and is capable of detecting, at regular intervals, the pressure that bears on the pressure sensor and accordingly, if the geometry of the tank is known, the weight of the liquid contained in the tank.

The weight variation between one measurement and the next, divided by the length of the measurement interval, is proportional to the reaction rate.

For example, when converting must into wine, the reaction rate is typically controlled so that alcohol production occurs at a rate on the order of 0.1 percent alcohol by volume per hour. A volume of 1000 liters of must, containing sugars for a potential 12 percent alcohol by volume, weighs 1089 kg and, after complete conversion of the sugars into alcohol, weighs 996.7 kg, with a weight variation of 92.3 kg. Developing 0.1 percent alcohol by volume therefore entails a weight loss of 0.77 kg, equal to approximately 0.70 ^{‰} of the initial weight.

The device according to the invention allows to detect the development of the reaction with a sensitivity of this order of magnitude also by virtue of some refinements, such as appropriate software of the PLC or PC which is suitable to eliminate or drastically reduce the effect of accidental measurements, for example by continuously computing the average of a significant number of consecutive measurements. Another refinement which has already been described consists in installing the pressure sensor so that it does not make direct contact with the liquid that is fermenting, which tends to form deposits on any membrane arranged between the sensor and the liquid, altering the measurement of the sensor.

It should be noted that the height h varies as the pressure to be detected varies; accordingly, this variable also must be considered when converting into weight of the liquid the electrical signal sent by the sensor to the control unit.

The detection device according to the present invention can be complemented by a second sensor, which is arranged at a higher level than the preceding one and is suitable to measure the density of the liquid, and by a temperature sensor. The measurements transmitted by these additional sensors to the processing unit allow, by means of an adapted software, to also control the density, volume and temperature of the liquid.

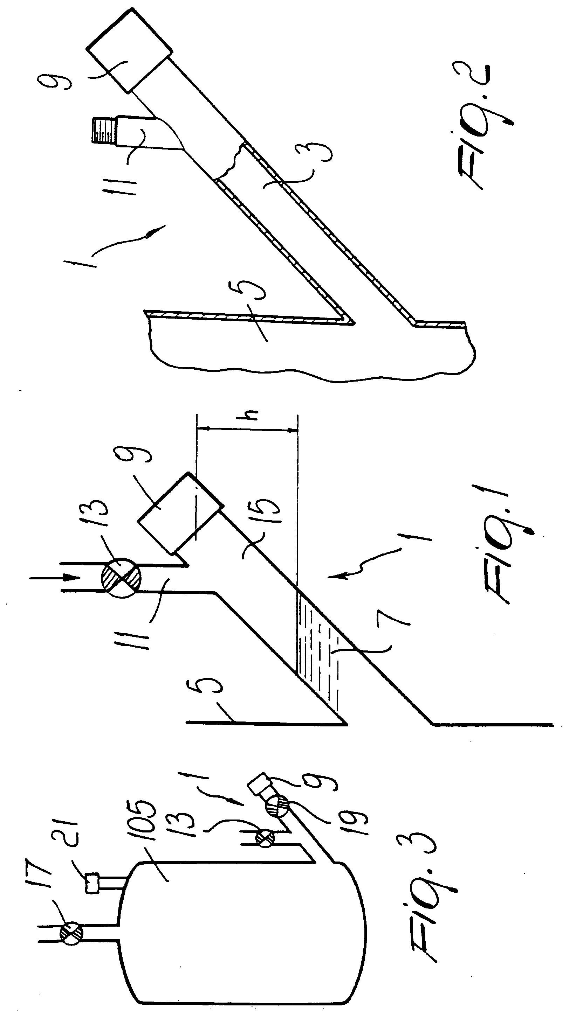

Figure 3 illustrates a particular application of the invention to a tank or pressure vessel 105.

In order to produce, by fermentation, beverages containing such amounts of carbon dioxide as to be sparkling or carbonated, part of the fermentation is performed in vessels which are capable of withstanding a considerable internal pressure, on the order of 6 bar.

One of the operating methods of these particular fermentation processes consists in allowing a first part of fermentation to occur while the pressure vessel 105 is connected to the atmosphere by opening an upper valve 17 until a preset alcohol percentage is reached, after which the upper valve 17 is closed and the carbon dioxide produced by subsequent fermentation partly saturates the fermenting liquid and partly generates an increase in pressure which helps to increase the amount of dissolved carbon dioxide.

With conventional systems, the time when the pressure vessel must be closed is determined by taking samples of the fermenting liquid and analyzing them with conventional methods. The system for detecting the development of fermentation according to the present invention can be applied advantageously to this particular type of fermentation process.

When the system for processing the measurements made with the sensor 9 reports that the preset alcohol content has been reached, a valve 19 arranged between the pressure vessel 105 and the sensor 9 is closed, optionally under the actuation of the detection system itself, and the upper valve 17 that connects the pressure vessel 105 to the atmosphere is closed directly thereafter. Conveniently but not necessarily, the same processing system (PLC or PC) can also act as vessel pressure control system by means of a sensor 21 to keep the pressure within the preset limits and optionally to start an apparatus adapted to slow or stop fermentation (for example an apparatus for cooling the fermenting liquid) by taking the pressure increase as an indicator of the development of fermentation.

Closure of the valve 19 is necessary to isolate the sensor 9 from the pressure inside the pressure vessel, so that the sensor 9 can work over a limited pressure range and thus has the sensitivity that is most suitable to detect the development of fermentation and at the same time is not damaged when, while the pressure vessel is closed and the valve 17 is closed, a significant pressure increase occurs in the pressure vessel.

In practice, it has been found that the invention achieves the intended aim and objects, a device for continuously detecting the development of reaction processes in a liquid having been provided which is capable of exactly indicating the development of the reaction in real time and without requiring intervention on the part of specialized personnel.

The detection device according to the invention is susceptible of numerous modifications and variations, all of which are within the scope of the inventive concept; all the details may furthermore be replaced with technically equivalent elements.

The materials used, as well as the dimensions, may of course be any according to the requirements and the state of the art.

## Claims

1. Device for continuously detecting the development of reaction processes in a liquid (7), comprising a tank (5, 105) which contains a liquid; characterized in that it comprises a means (3, 9, 15) for detecting the weight of the liquid (7), said means being associated with a unit for processing the measurement of said weight, said unit being adapted to determine the weight variation over time in order to control the development of a reaction process which causes a change in the weight of said liquid (7).

2. Detection device according to claim 1, characterized in that said weight detection means comprises a pressure sensor (9).

3. Detection device according to claim 1 or 2, characterized in that said pressure sensor (9) is located proximate to the base of the tank (5).

4. Detection device according to one or more of the preceding claims, characterized in that said pressure sensor (9) is applied to a tubular body (3) which is associated with the tank (5) so as to form an air pocket (15) between the pressure sensor and the surface of the liquid contained in the tank.

5. Detection device according to one or more of the preceding claims, characterized in that said tubular body (3) has a coupling (11) for a valve means (13) which is adapted to allow to introduce a jet of washing fluid adapted to wash the tubular body portion affected by said liquid (7) without striking said pressure sensor (9).

6. Detection device according to one or more of the preceding claims, characterized in that it comprises a second sensor connected to said processing unit, said second sensor being located at a higher level than the pressure sensor and being adapted to detect the density of the liquid.

7. Detection device according to one or more of the preceding claims, characterized in that it comprises a third sensor connected to said processing unit and adapted to detect the temperature of the liquid.

8. Pressure vessel for fermenting a liquid, characterized in that it comprises an upper valve (17) adapted to selectively connect and isolate the inside of the pressure vessel with respect to the atmosphere, a device (3, 9, 15) for continuously detecting the development of the liquid fermentation processes, said device comprising a means for detecting the weight of the liquid, associated with a unit for processing the measurement of said weight and suitable to determine weight variation over time in order to control the development of the fermentation of the liquid (7) which causes a variation in the weight of said liquid, at least when said upper valve is open.

9. Pressure vessel according to claim 8, characterized in that it comprises a second valve (19) adapted to isolate said weight detection means (9) from said inside of said pressure vessel.

10. Pressure vessel according to claim 8, characterized in that it comprises a means (21) for detecting the internal pressure and adapted to operate at least when said weight detection means (9) is isolated from said interior of said pressure vessel.

11. Pressure vessel according to one or more of claims 8-10, characterized in that it comprises a unit for processing the measurement of said weight, said unit being suitable to determine the weight variation over time in order to control the development of said fermentation, which causes a variation in the weight of said liquid.

12. Pressure vessel according to claim 11, characterized in that said upper valve (17) and said pressure detection means (9) are connected to said processing unit.
